# EUROPEAN PATENT APPLICATION

(11) **EP 4 610 357 A1**
(43) Date of publication of application: **03.09.2025**
(21) Application number: 23898357.1
(22) Date of filing: 30.11.2023
(51) Int. Cl.: C12N 15/52, C12N 15/70, C12N 15/74, C12N 9/04, C12N 9/16, C12P 7/18, C12P 7/52, C12N 9/88

(54) **MICROORGANISM SIMULTANEOUSLY PRODUCING 1,3-PROPANEDIOL AND 3-HYDROXYPROPIONIC ACID, AND USE THEREOF**

(30) Priority: 30.11.2022 KR 20220165039; 30.11.2022 KR 20220165127
(71) Applicant: LG CHEM, LTD., Seoul 07336 (KR)
(72) Inventor: SUNG, Min Kyung, Daejeon 34122 (KR); YEOM, Sujin, Daejeon 34122 (KR); KANG, Donggyun, Daejeon 34122 (KR); KIM, Jae Hyung, Daejeon 34122 (KR)
(74) Representative: Plasseraud IP
(86) International application number: PCT/KR2023/019618
(87) International publication number: WO 2024/117834

(57) **Abstract**

The present specification relates to a microorganism into which a GPD gene, a GPP gene, and dhaB, gdrAB, aldH and/or yqhD genes are introduced, and/or use thereof, the microorganism being capable of simultaneously producing 1,3-PDO and 3-HP.

## Description

### [TECHNICAL FIELD]

### Cross-reference to related application(s)

The present application claims the benefit of the priority based on Korean patent application No. 10-2022-0165039 filed on November 30, 2022 and Korean patent application No. 10-2022-0165127 filed on November 30, 2022, and the entire contents disclosed in the documents of the corresponding Korean patent applications are incorporated as a part of the present description.

The present description relates to a microorganism to which a gene encoding glycerol-3-phosphate dehydrogenase (GPD) (hereinafter, GPD gene) and/or a gene encoding glycerol-3-phosphate phosphatase (GPP) (hereinafter, GPP gene), dhaB, gdrAB, aldH, and/or yqhD genes are introduced and/or uses thereof.

### [BACKGROUND ART]

1,3-propanediol (1,3-PDO), an aliphatic diol, is an organic chemical substance with a relatively simple structure, also called trimethylene glycol or 1,3-dihydroxypropane or the like, and is used for various uses including polymers, cosmetics and lubricants. Polytrimethylene terephthalate (PTT), which is produced by a polymerization reaction of 1,3-PDO and terephthalic acid and commercialized, is greatly spotlighted due to more excellent characteristics compared to polyester and nylon in terms of elasticity and optical stability and the like.

3-Hydroxypropionic Acid (3-HP) is a platform compound which can be converted into various chemicals such as acrylic acid, methyl acrylate, acrylamide and the like, and has been actively studied in the academia and industry since it was selected as Top12 Value-added Bio-chemical by U.S. Department of Energy in 2004.

In order to simultaneously biosynthesize 1,3-PDO and 3-HP, which are high value-added monomers, various microorganisms have been developed. However, conventional strains have a low titer or use a method for mixed culture of two kinds of microorganisms and the like, and there is a lack of research on biosynthesizing 1,3-PDO and 3-HP from glucose or glycerol simultaneously, and thus, there is a limitation in applying them to an actual process.

### [DISCLOSURE]

### [TECHNICAL PROBLEM]

Under the above background, the present inventors have repeatedly conducted research on microorganisms to biosynthesize 1,3-PDO and 3-HP simultaneously, and as a result, it has been confirmed that a microorganism to which a gene encoding glycerol-3-phosphate dehydrogenase (GPD), a gene encoding glycerol-3-phosphate phosphatase (GPP), dhaB, gdrAB, aldH, and yqhD are introduced produces 1,3-PDO and 3-HP from glucose and/or glycerol simultaneously, thereby completing the present invention.

One embodiment of the present description provides a microorganism, comprising at least one (2 kinds or more, 3 kinds or more, or 4 kinds) gene selected from the group consisting of dhaB, gdrAB, aldH and yqhD (a first recombinant microorganism). The first recombinant microorganism may have production ability (for example, simultaneous production ability) of 1,3-PDO and 3-HP from glycerol.

The microorganism may further comprise at least one gene selected from the group consisting of a gene encoding glycerol-3-phosphate dehydrogenase (GPD) (GPD gene) and a gene encoding glycerol-3-phosphate phosphatase (GPP) (GPP gene) (a second recombinant microorganism). The second recombinant microorganism may have production ability (for example, simultaneous production ability) of 1,3-PDO and 3-HP from glucose.

Another embodiment provides a recombinant vector comprising at least one (2 kinds or more, 3 kinds or more, or 4 kinds) selected from the group consisting of dhaB, gdrAB, aldH and yqhD (a first recombinant vector).

The recombinant vector may further comprise at least one gene selected from the group consisting of GPD gene and GPP gene (a second recombinant vector).

The first recombinant vector and the second recombinant vector may have a use of producing 1,3-PDO and/or 3-HP. In one embodiment, the first recombinant vector may be a recombinant vector for producing 1,3-PDO and/or 3-HP from glycerol. In another embodiment, the second recombinant vector may be a recombinant vector for producing 1,3-PDO and/or 3-HP from glucose.

Other embodiment provides a composition for producing 1,3-PDO and/or 3-HP, comprising the microorganism and/or recombinant vector. In one embodiment, a composition for producing 1,3-PDO and/or 3-HP from glycerol, comprising the first recombinant microorganism, the first recombinant vector, or both of them is provided. In another embodiment, a composition for producing 1,3-PDO and/or 3-HP from glucose, comprising the second recombinant microorganism, the second recombinant vector, or both of them is provided.

Other embodiment provides a method for producing 1,3-PDO and/or 3-HP, comprising culturing the microorganism.

Other embodiment provides a fermented composition, which is obtained by fermenting a microorganism having producing activity of the 1,3-PDO and 3-HP, and comprises 1,3-PDO and 3-HP.

### [TECHNICAL SOLUTION]

One embodiment of the present description provides a microorganism, comprising at least one (2 kinds or more, 3 kinds or more, or 4 kinds) selected from the group consisting of dhaB, gdrAB, aldH and yqhD.

The microorganism may further comprise at least one gene selected from the group consisting of a gene encoding glycerol-3-phosphate dehydrogenase (GPD) (GPD gene) and a gene encoding glycerol-3-phosphate phosphatase (GPP) (GPP gene) (a second recombinant microorganism).

The microorganism may comprise all of the dhaB, gdrAB, aldH and yqhD genes, but not limited thereto.

The microorganism may comprise all of the GPD gene and GPP gene, but not limited thereto.

The microorganism may produce the 1,3-propanediol (1,3-PDO) and/or 3-hydroxypropionic acid (3-HP), and may produce 1,3-PDO and 3-HP simultaneously, but not limited thereto.

The microorganism may have production activity of the 1,3-propanediol (1,3-PDO) and/or 3-hydroxypropionic acid (3-HP), and may have simultaneously producing activity of the 1,3-propanediol (1,3-PDO) and 3-hydroxypropionic acid (3-HP), but not limited thereto.

The microorganism provided in the present description may produce 1,3-propanediol (1,3-PDO) and 3-hydroxypropionic acid (3-HP) from glucose and/or glycerol, but not limited thereto.

Another embodiment provides a composition for producing 1,3-PDO and/or 3-HP, comprising the microorganism and/or recombinant vector provided in the present description.

Other embodiment
provides a recombinant vector comprising at least one (2 kinds or more, 3 kinds or more, or 4 kinds) selected from the group consisting of dhaB, gdrAB, aldH and yqhD.

The recombinant vector may further comprise at least one gene selected from the group consisting of GPD gene and GPP gene (a second recombinant vector).

The first recombinant vector may comprise dhaB, gdrAB, aldH and yqhD genes, but not limited thereto.

The second recombinant vector may comprise GPD gene and GPP gene, but not limited thereto.

Other embodiment provides a method for producing 1,3-PDO and/or 3-HP, comprising culturing the microorganism provided in the present description.

Hereinafter, the present application will be described in more detail.

In the present description, "microorganism" means a very small organism that cannot be seen with naked eyes. In the present description, the microorganism may have ability to synthesize an organic compound, and for example, it may have ability to synthesize 1,3-propanediol (1,3-PDO) and/or 3-hydroxypropionic acid (3-HP) (or, 1,3-PDO production ability and/or 3-HP production ability). The microorganism according to one embodiment itself may be a strain genetically engineered to produce organic compounds, or to have production ability of organic compounds or to enhance production ability of organic compounds by comprising a gene involved in producing organic compounds. Production of the organic compounds includes production within the strain, production within a cell and secretion outside the cell, or a combination thereof.

The microorganism of the present description may have enhanced activity of the introduced gene, as at least one gene selected from the group consisting of a gene encoding glycerol-3-phosphate dehydrogenase (GPD) (GPD gene), a gene encoding glycerol-3-phosphate phosphatase (GPP) (GPP gene), dhaB, gdrAB, aldH and yqhD is introduced, but not limited thereto.

The microorganism of the present description may further comprise a characteristic of weakened or inactivated activity of at least one gene selected from the group consisting of yqhD, glpK, ldhA, ack-pta, gldA and ptsG, but not limited thereto.

The microorganism of the present description may further comprise at least one characteristic selected from the group consisting of the following (a) and (b), but not limited thereto:
(a) weakened or inactivated activity of at least one gene selected from the group consisting of yqhD, glpK, ldhA, ack-pta, gldA and ptsG; and
(b) enhanced activity of at least one gene selected from the group consisting of galP and glk.

The characteristics of (a) and (b) may be characteristics of a microorganism before at least one gene selected from the group consisting of a gene encoding glycerol-3-phosphate dehydrogenase (GPD) (GPD gene), a gene encoding glycerol-3-phosphate phosphatase (GPP) (GPP gene), dhaB, gdrAB, aldH and yqhD is introduced to the microorganism of the present description, and the corresponding characteristics may be maintained, or not maintained after the gene is introduced, but not limited thereto.

The microorganism of the present description may be an Escherichia sp. microorganism (for example, E. coli, E. albertii, E. fergusonii, E. hermannii, and E. marmotae), a Klebsiella sp. microorganism (for example, K. pneumoniae, K. aerogenes, K. granulomatis, K. grimontii, K. huaxiensis) and/or a Lactobacillus sp. microorganism (for example, L. reuteri, L. acetotolerans, L. acidophilus, L. thermophilus), but not limited thereto.

The microorganism provided in the present description may have 1,3-propanediol (1,3-PDO) production ability and/or 3-HP production ability, but not limited thereto.

In the present description, " comprises a gene" may mean not only comprising a gene by a subject (for example, microorganism) of comprising but also introducing and comprising a foreign gene to the subject.

In the present description, " have 1,3-propanediol (1,3-PDO) production ability" may mean a cell and/or a microorganism naturally having 1,3-PDO production ability, or a microorganism in which 1,3-PDO production ability is given to a parent strain having no 1,3-PDO production ability. It may be used to mean the case in that the microorganism has 1,3-PDO production ability by introducing the mutation according to one embodiment and/or the case in that the microorganism has 1,3-PDO production ability by introducing the mutation according to one embodiment into a microorganism having no 1,3-PDO production ability. For example, the Escherichia sp. microorganism having 1,3-PDO production ability may mean a natural microorganism itself or an Escherichia sp. microorganism to have improved 1,3-PDO production ability by inserting a foreign gene related to the 1,3-PDO production mechanism or enhancing or inactivating activity of an intrinsic gene.

In the present description, " have 3-HP production ability" may mean a cell and/or a microorganism naturally having 3-HP production ability or a microorganism in which 3-HP production ability is given to a parent strain having no 3-HP production ability. It may be used to mean the case in that the microorganism has 3-HP production ability by introducing the mutation according to one embodiment and/or the case in that the microorganism has 3-HP production ability by introducing the mutation according to one embodiment into a microorganism having no 3-HP production ability. For example, the Escherichia sp. microorganism having 3-HP production ability may mean a natural microorganism itself or an Escherichia sp. microorganism to have improved 3-HP production ability by inserting a foreign gene related to the 3-HP production mechanism or enhancing or inactivating activity of an intrinsic gene.

In one embodiment, the microorganism may be a strain which has 1,3-PDO production ability and/or 3-HP production ability or is genetically engineered to have 1,3-PDO production ability and/or 3-HP production ability. The production of 1,3-PDO and/or production of 3-HP include production within the strain, production within a cell and secretion outside the cell, or a combination thereof.

The microorganism (or recombinant cell) may additionally comprise a mutation to increase 1,3-PDO production and/or 3-HP production, and the location of the mutation and/or the type of gene and/or protein subject to the mutation may be included without limitation as long as it increases 1,3-PDO production and/or 3-HP production. The microorganism may be used without limitation as long as it is a cell capable of transformation.

In the present application, in order to distinguish a microorganism before the mutation according to one embodiment is introduced from a microorganism in which 1,3-PDO production ability and/or 3-HP production ability increase, or 1,3-PDO production ability and/or 3-HP production ability are given as the mutation according to one embodiment is introduced, the microorganism before the mutation according to one embodiment is introduced may be expressed as a host microorganism (or parent strain).

In the present description, "glycerol-3-phosphate dehydrogenase (GPD)" refers to a polypeptide having enzymatic activity to catalyze conversion from dihydroxyacetone phosphate (DHAP) to glycerol-3-phosphate (G3P). Glycerol-3-phosphate dehydrogenase may be NADH, NADPH or FAD-dependent. Glycerol-3-phosphate dehydrogenase, regardless of its origin, is included in the scope of the present application as long as it is an enzyme having enzymatic activity to catalyze conversion from DHAP to G3P, and GPD1 or GPD2 may be exemplified as genes encoding it.

In the present description, "glycerol-3-phosphate phosphatase (GPP)" refers to a polypeptide having enzymatic activity to catalyze conversion from glycerol-3-phosphate (G3P) to glycerol. Glycerol-3-phosphate phosphatase, regardless of its origin, is included in the scope of the present application as long as it is an enzyme having enzymatic activity to catalyze conversion from G3P to glycerol, and GPP1 or GPP2 may be exemplified as genes encoding it.

In the present description, " glycerol dehydratase" or " diol dehydratase" may mean a polypeptide having enzymatic activity to catalyze conversion from glycerol to 3-hydroxypropionaldehyde (3-HPA). Glycerol dehydratase or diol dehydratase may be coenzyme B12 dependent or independent. The enzyme may be derived from Klebsiella pneumoniae, Citrobacter freundii, Clostridium pasteurianum, Salmonella typhimurium, Klebsiella oxytoca, and/or Clostridium butyricum, and the like, but not limited thereto. The enzyme, regardless of its origin, may be included in the scope of the present application as long as it is an enzyme having enzymatic activity to catalyze conversion from glycerol to 3-hydroxypropionaldehyde (3-HPA), and for example, a gene encoding it may be dhaB.

In the present description, " glycerol dehydratase reactivase" may mean an enzyme which acts to maintain catalytic activity by activating what is irreversibly inactivated while glycerol dehydratase acts. As glycerol dehydratase reactivase, any one generally known may be used, and for example, it may be derived from a Klebsiella sp. (for example, Klebsiella pneumonia), a Citrobacter sp., a Lactobacillus sp., a Salmonella sp. strain, or the like, but not limited thereto. Glycerol dehydratase reactivase may be DhaFG, GdrAB, DdrAB, or the like, and for example, genes encoding it may be dhaFG, gdrAB, and/or ddrAB.

In the present description, " yqhD" is a gene encoding alcohol dehydrogenase, and encodes an enzyme which converts 3-hydroxypropanal to 1,3-PDO when glycerol is degraded into 3-hydroxypropanal and water by glycerol dehydratase.

In the present description, " aldehyde dehydrogenase" is an enzyme which catalyzes oxidation of aldehyde. The enzyme can convert aldehyde (R-C(=O)-H) to carboxylic acid (R-C(=O)-O-H). The enzyme may be encoded by aldH gene, and the gene may be aldH (GenBank Accession no. U00096.3; EaldH) gene derived from an Escherichia coli or E. coli K12 MG1655 18-3 cell line, puuC gene derived from Klebsiella pneumonia (K. pneumonia), and/or KGSADH gene derived from Azospirillum brasilense, but not limited thereto. The aldehyde hydrogenase protein and gene encoding it may comprise a mutation of the gene and/or amino acid sequence within the range of maintaining activity to produce 3-HP from 3-HPA.

In the present description, " enhancing activity" or " enhanced activity" may not only comprise deriving effects beyond the original function as the activity of protein itself is newly introduced or increases, but also mean a condition under which the activity of the microorganism is increased after engineering is conducted, compared to the activity of the microorganism before engineering such as increase in gene expression, increase in intrinsic gene activity, amplification of an intrinsic gene from an internal or external factor, deletion of an inhibition regulatory factor of the gene expression, increase in gene copy number, gene introduction from the outside, modification of an expression regulatory sequence, and/or promoter replacement or modification and increase in enzymatic activity by intragenic mutation, and the like, is conducted.

In the present description, enhancing activity of protein and/or enzyme (for example, GPD, GPP, dhaB, gdrAB, aldH and/or yqhD) may be achieved by application of various methods well known in the art. To the method for enhancing or increasing the activity of the protein and/or enzyme, various methods well known in the art can be applied. For example, it is not limited thereto, but a method for inserting a polynucleotide comprising a sequence encoding the corresponding enzyme (or protein) to chromosome additionally, a method for increasing a copy number of a sequence encoding enzymes (or proteins) by a method for introducing the polynucleotide into a vector system and the like, a method for replacing a promoter capable of expressing the polynucleotide with a strong promoter, and a method for introducing a mutation into a promoter may be included, and there is a method for mutating into an enzyme (or protein) with strong activity by gene mutation and the like. The method for inserting a polynucleotide (for example, GPD gene, GPP gene, dhaB, gdrAB, aldH and/or yqhD) to host cell genome (chromosome) may be performed by appropriately selecting a known method by those skilled in the art, and for example, it may be performed using (a) RNA-guided endonuclease (for example, Cas9 protein, etc.), a gene encoding thereof, or a vector comprising the gene; and (b) a mixture (a mixture of RNA-guided endonuclease protein and guide RNA, etc.) or complex (for example, at least one selected from the group consisting of ribonucleic acid fusion protein (RNP), a recombinant vector (for example, a vector comprising a gene encoding RNA-guided endonuclease and DNA encoding guide RNA together, etc.) and the like), comprising guide RNA (for example, single guide RNA (sgRNA), etc.), DNA encoding thereof, or a vector comprising the DNA, but not limited thereto.

In the present description, " weakened activity" may mean the case in that the activity level of protein and/or enzyme (for example, GPD, GPP, yqhD, glpK, ldhA, ack-pta, gldA and/or ptsG) is reduced in a cell, compared to a host cell (or parent strain) such as a wild-type strain or a strain before modified or the like. The weakening is a concept including the case in that the activity of enzyme (or protein) itself is reduced compared to the activity of enzyme which a microorganism originally has due to mutation of a gene encoding enzyme (or protein), and the case in that the expression level of the enzyme (or protein) is reduced due to inhibition of transcription and/or inhibition of translation of a gene encoding thereof and the like, and thus the total enzyme (or protein) activity level is lower than a wilt-type strain or a strain before modified in a cell, and also a combination thereof.

In addition, in the present description, " inactivating" may mean a condition that expression of a gene encoding protein and/or enzyme (for example, GPD gene, GPP gene, yqhD, glpK, ldhA, ack-pta, gldA and/or ptsG) is not done at all, or it is expressed, but it has no activity.

In the present description, weakening or inactivating activity of protein and/or enzyme (for example, GPD, GPP, yqhD, glpK, ldhA, ack-pta, gldA and/or ptsG), may be achieved by application of various methods well known in the art. For example, there are a method for replacing a gene encoding the enzyme with a gene mutated to reduce the enzyme (or protein) activity including the case in that the activity of enzyme (or protein) is completely removed; a method for introducing a mutation into an expression regulatory sequence (for example, promoter) of a gene on chromosome encoding the enzyme (or protein); a method for replacing an expression regulatory sequence of a gene encoding the enzyme (or protein) with a sequence with weak activity, or no activity; a method for deleting all or some of genes on chromosome encoding the enzyme (or protein); a method for introducing antisense oligonucleotide (for example, antisense RNA) which complementarily binds to transcriptome of the gene on chromosome to inhibit translation from the mRNA to enzyme (or protein); a method for making attachment of ribosome impossible by artificially adding a complementary sequence to an SD sequence in front of the SD sequence of a gene encoding the enzyme (or protein) to form a secondary structure; and/or an RTE (Reverse transcription engineering) method of adding a promoter to be reverse transcribed to the 3' end of the ORF (open reading frame) of the corresponding sequence, and the like, and it may be achieved by a combination thereof, but not limited thereto.

In the present description, reduction of gene expression may mean that the expression level of a gene is reduced than the expression level of a host cell before mutated (parent strain) and/or a wild-type, and may include the case in that expression is completely inhibited and therefore expression is not achieved. When expression of a gene is to be reduced in a microorganism, an initiation codon of gene translation may be substituted to reduce its expression, or it may be genetically engineered to reduce the expression level of a gene present conventionally. A method for modifying a sequence for regulation of expression may be performed by inducing a mutation on an expression regulatory sequence by deletion, insertion, non-conservative or conservative substitution, or a combination thereof in the nucleic acid sequence of the expression regulatory sequence, or may be performed by a method for replacing a promoter with a further weaker promoter and the like. The expression regulatory sequence includes a promoter, an operator sequence, a sequence encoding a ribosome binding site, and a sequence regulating termination of transcription and translation, but not limited thereto. When a gene reducing expression is present in a microorganism to be mutated, expression of the gene may be reduced, for example, by substituting a native promoter operating expression of the gene with a weak promoter, or introducing a mutation. Furthermore, expression of the gene may be reduced by deleting all or some of conventional genes.

In the present description, " vector" refers to any vehicle for cloning and/or transferring a base to a host cell. The vector may be a replicon capable of bringing replication of a fragment combined as another DNA fragment is combined. " Replicon" may mean any genetic unit (for example, plasmid, phage, cosmid, chromosome, virus), which functions as a self-unit of DNA replication in vivo, that is, can be replicated by its own control. In the present invention, the vector is not particularly limited as long as it is replicable in a host, and any vector known in the art may be used. The vector used for construction of the recombinant vector may be a plasmid, cosmid, virus, and/or bacteriophage in a natural state or a recombinant state. For example, as the phage vector or cosmid vector, pWE15, M13, λ EMBL3, λ EMBL4, λ FIXII, λ DASHII, λ ZAPII, λ gt10, λ gt11, Charon4A, and/or Charon21A and the like may be used, and as the plasmid vector, pDZ vector, pBR-based, pUC-based, pBluescriptII-based, pGEM-based, pTZ-based, pCL-based, and/or pET-based and the like may be used. The available vector is not particularly limited, and a known expression vector may be used. In one embodiment, the vector may be that a gene delivered by being inserted into the vector is irreversibly fused into genome of a host cell, thereby allowing gene expression to continue stably for a long period of time within a cell. Such a vector may comprise a transcription and translation expression regulatory sequence which allows the corresponding gene to be expressed in a selected host. As the expression regulatory sequence, any operator sequence for regulating transcription, and/or a sequence for regulating termination of transcription and translation may be included. In one embodiment, the initiation codon and termination codon may be generally considered a part of the nucleic acid sequence encoding target protein, and should be functional in a subject when a gene construct is administered, and may be in frame with the coding sequence. In addition, in case of a replicable expression vector, it may comprise an origin of replication. In addition, it may appropriately comprise an enhancer, a non-translated region at the 3' end of a target gene, a selection marker (for example, antibiotic resistance marker), and/or a replicable unit, and the like. The vector may be self-replicated or integrated into host genomic DNA.

According to one embodiment, each element in the vector should be operably linked to each other, and linking of these element sequences may be performed by ligation (linking) at a convenient restriction enzyme site, and when such a site is not present, it may be performed using a synthetic oligonucleotide adaptor or a linker according to a common method.

In the present description, " transduction" refers to a phenomenon in which bacterial DNA is moved to another bacterium mediating bacteriophage, and is one of methods of horizontal gene transfer (HGT). A bacteriophage capable of transduction in this way is called " transduction particle" or " transduction phage" , and a cell receiving DNA from another bacterium is called " transductant" .

In the present description, " transformation" is introducing a gene into a host cell to express it in a host cell, and the transformed gene may be comprised as long as it can be expressed in a host cell, without limiting to whether it is inserted in chromosome of the host cell or located outside the chromosome. In addition, the gene comprises DNA and/or RNA encoding target protein. As long as the gene can be introduced into a host microorganism and expressed, its form to be introduced is not limited. For example, the gene may be introduced into a host microorganism in an expression cassette form which is a gene structure comprising all elements required for being expressed by itself. The expression cassette may commonly comprise an expression regulatory element such as a promoter, a transcription termination signal, a ribosome binding site and/or a translation termination signal and the like, which are operably linked to the gene. The expression cassette may be in an expression vector form capable of self-replication. In addition, the gene may be introduced to a host cell in a form of itself and be operably linked to a sequence required for expression in the host cell.

In the present description, " promoter" means a non-translated nucleic acid sequence of upstream of a coding region, which comprises a binding site for polymerase and has transcription initiation activity into mRNA of a promoter downstream gene, that is, a DNA region to which polymerase binds to initiate transcription of a gene, and may be located in the 5' region of the mRNA transcription initiation region.

In one embodiment, the microorganism provided in the present description can produce 1,3-propanediol (1,3-PDO) and/or 3-hydroxypropionic acid (3-HP) by additionally comprising a constitutive promoter, and GPD gene, GPP gene, dhaB, gdrAB, aldH and/or yqhD which are operably linked thereto, but not limited thereto. The constitutive promoter can induce expression of the operably linked gene without a separate inducer. For example, the constitutive promoter may be J23-based (for example, the promoter is J23100, J23101, J23102, J23103, J23104, J23105, J23106, J23107, J23109, J23111, J23112, J23113, J23114, J23115, J23116, J23117, and/or J23118), but not limited thereto, and may be selected and used without limitation as needed within the range of the purpose of producing 1,3-PDO and/or 3-HP by those skilled in the art. At least one (2 kinds or more, 3 kinds or more, or 4 kinds) gene selected from the group consisting of GPD gene, GPP gene, dhaB, gdrAB, aldH and yqhD, in which the constitutive promoter is introduced, may be cloned into a vector, respectively, and introduced into a cell (or microorganism). Otherwise, the four genes may be cloned into one vector and introduced into a cell (or microorganism).

In the present description, " productivity" may mean the produced amount per hour, but not limited thereto.

The microorganism provided in the present description may have 1,3-PDO productivity of 0.1 g/L/hr or more, 0.5 g/L/hr or more, 1 g/L/hr or more, 1.2 g/L/hr or more, 1.3 g/L/hr or more, 1.4 g/L/hr or more, 1.45 g/L/hr or more, 1.6 g/L/hr or more, 1.9 g/L/hr or more, 2 g/L/hr or more, or 2.1 g/L/hr or more, for example, 1.45 g/L/hr or 2.1 g/L/hr, and the upper limit of the 1,3-PDO productivity is not particularly limited, and for example, it may have 1,3-PDO productivity of 100 g/L/hr or less or 10 g/L/hr or less, but not limited thereto.

The microorganism provided in the present description may have 3-HP productivity of 0.1 g/L/hr or more, 0.5 g/L/hr or more, 1 g/L/hr or more, 1.2 g/L/hr or more, 1.3 g/L/hr or more, 1.4 g/L/hr or more, 1.45 g/L/hr or more, 1.6 g/L/hr or more, 1.9 g/L/hr or more or 2 g/L/hr or more, for example, 1.45 g/L/hr or 2 g/L/hr, and the upper limit of the 3-HP productivity is not particularly limited, and for example, it may have 3-HP productivity of100 g/L/hr or less or 10 g/L/hr or less, but not limited thereto.

The present description provides a recombinant vector, comprising at least one gene (2 kinds or more, 3 kinds or more, or 4 kinds) selected from the group consisting of dhaB, gdrAB, aldH and yqhD.

The present description provides a recombinant vector, comprising at least one (2 kinds or more, 3 kinds or more, or 4 kinds) selected from the group consisting of dhaB, gdrAB, aldH and yqhD (the first recombinant vector).

The recombinant vector may further comprise at least one gene selected from the group consisting of GPD gene and GPP gene (the second recombinant vector).

A microorganism in which the recombinant vector is introduced may produce 1,3-PDO and/or 3-HP, and may simultaneously produce 1,3-PDO and/or 3-HP, but not limited thereto.

A microorganism in which the recombinant vector is introduced may have 1,3-PDO and/or 3-HP production activity, and may have simultaneous production activity of 1,3-PDO and/or 3-HP, but not limited thereto.

The present description provides a method for producing 1,3-PDO and/or 3-HP, comprising culturing the microorganism (specifically, comprising culturing a microorganism, comprising at least one (2 kinds or more, 3 kinds or more, or 4 kinds) selected from the group consisting of dhaB, gdrAB, aldH and yqhD and/or at least one gene selected from the group consisting of a gene encoding glycerol-3-phosphate dehydrogenase (GPD) (GPD gene) and a gene encoding glycerol-3-phosphate phosphatase (GPP) (GPP gene).

Other embodiment provides a method for producing 1,3-PDO and/or 3-HP from glycerol, comprising culturing the first recombinant microorganism. Other embodiment provides a method for producing 1,3-PDO and/or 3-HP from glucose, comprising culturing the second recombinant microorganism.

The culturing a microorganism (specifically, the first recombinant microorganism and/or the second recombinant microorganism) may comprise reacting the microorganism with a substrate. The substrate may be glycerol and/or glucose, but not limited thereto.

The method for producing 1,3-PDO and/or 3-HP may further comprise recovering 1,3-PDO and/or 3-HP from the cultured medium or microorganism, but not limited thereto.

In one embodiment, the culturing may comprise primary culturing and/or primary culturing and secondary culturing, but not limited thereto.

In one embodiment, the culturing may comprise culturing by inoculating to an LB medium;
culturing by inoculating to an M9 medium; and/or
culturing by inoculating to an MR medium, but not limited thereto.

Culturing may be performed using the culturing by inoculating to an LB medium as primary culture, and the culturing by inoculating to an M9 medium and/or an MR medium as secondary culture, but not limited thereto.

The 1,3-PDO productivity of the method for producing 1,3-PDO and/or 3-HP may be 0.1 g/L/hr or more, 0.5 g/L/hr or more, 1 g/L/hr or more, 1.2 g/L/hr or more, 1.3 g/L/hr or more, 1.4 g/L/hr or more, 1.45 g/L/hr or more, 1.6 g/L/hr or more, 1.9 g/L/hr or more, 2 g/L/hr or more, or 2.1 g/L/hr or more, for example, 1.45 g/L/hr or 2.1 g/L/hr, and the upper limit of the 1,3-PDO productivity is not particularly limited, and for example, it may be 100 g/L/hr or less or 10 g/L/hr or less, but not limited thereto.

The 3-HP productivity of the method for producing 1,3-PDO and/or 3-HP may be 0.1 g/L/hr or more, 0.5 g/L/hr or more, 1 g/L/hr or more, 1.2 g/L/hr or more, 1.3 g/L/hr or more, 1.4 g/L/hr or more, 1.45 g/L/hr or more, 1.6 g/L/hr or more, 1.9 g/L/hr more or 2 g/L/hr or more, for example, 1.45 g/L/hr or more or 2 g/L/hr, and the upper limit of the 3-HP productivity is not particularly limited, and for example, it may be 100 g/L/hr or less or 10 g/L/hr or less, but not limited thereto.

The culturing may be conducted according to the appropriate medium and culture conditions known in the art, and should satisfy requirements of a specific strain, and may be appropriately modified by those skilled in the art.

The culture method may include for example, batch culture, continuous culture, fed-batch culture, or a combined culture thereof, but not limited thereto.

According to one embodiment, various carbon sources, nitrogen sources and trace element components may be comprised in the medium, and the recombinant cell may be cultured while adjusting the temperature and/or pH and the like in a common medium containing suitable carbon sources, nitrogen sources, amino acids, vitamins and the like. As the available carbon sources, sugar and carbohydrates such as glucose, sucrose, lactose, fructose, maltose, starch and/or cellulose and oils and fats such as soybean oil, sunflower oil, castor oil, and/or coconut oil, and the like, fatty acids such as palmitic acid, stearic acid, and/or linoleic acid, alcohols such as glycerol and/or ethanol, and organic acids such as acetic acid are included. These substances may be used individually or in a mixture, but not limited thereto. As the available nitrogen sources, peptone, yeast extract, gravy, malt extract, corn steep liquor, soybean meal and urea or inorganic compounds, for example, ammonium sulfate, ammonium chloride, ammonium phosphate, ammonium carbonate and ammonium nitrate may be included. The nitrogen sources may be also used individually or in a mixture, but not limited thereto. As the available sources of supply of phosphorus, potassium dihydrogen phosphate or dipotassium hydrogen phosphate or corresponding sodium-containing salts may be included, but not limited thereto. In addition, the medium may contain a metal salt such as magnesium sulfate or iron sulfate required for growth, but not limited thereto. In addition, essential growth substances such as amino acids and vitamins may be comprised. Moreover, precursors suitable for the medium may be used. The medium or individual components may be added to the culture solution in a fed-batch or continuous method by an appropriate method during the culture process, but not limited thereto.

The separating and/or recovering 1,3-PDO and/or 3-HP from the cultured microorganism (or recombinant cell) and/or culture medium may be collecting a desired substance (for example, 1,3-PDO and/or 3-HP) from the medium, culture solution, or microorganism using an appropriate method known in the art. For example, methods of centrifugation, filtration, extraction, spraying, drying, evaporation, precipitation, crystallization, electrophoresis, fractional dissolution (for example, ammonium sulfate precipitation), and/or chromatography (for example, ion exchange, affinity, hydrophobic, liquid, and size exclusion) and the like may be used, but not limited thereto. The culture medium may mean a medium in which a microorganism (or recombinant cell) is cultured.

In one embodiment, the method for producing 1,3-PDO and/or 3-HP may comprise purifying 1,3-PDO and/or 3-HP before, at the same time with, or after the separating and/or recovering additionally.

In the present description, "fermented composition" means a composition prepared by adding a strain and/or a microorganism to a specific material through a process of culturing and/or fermenting.

The present description provides a fermented composition, which is obtained by fermenting a microorganism having producing activity of 1,3-PDO and 3-HP, and comprises 1,3-PDO and/or 3-HP.

The fermented composition may comprise 1,3-PDO at a concentration of 10 g/L or more, 15 g/L or more, 20 g/L or more, 25 g/L or more, 50 g/L or more, 55 g/L or more, 60 g/L or more, 65 g/L or more, 70 g/L or more, 75 g/L or more, 80 g/L or more or 81 g/L or more, for example, 26 g/L or 81 g/L, and the upper limit of the 1,3-PDO concentration is not particularly limited, and for example, it may comprise it at a concentration of 1000 g/L or less, or 100 g/L or less, but not limited thereto.

The fermented composition may comprise 3-HP at a concentration of 10 g/L or more, 15 g/L or more, 20 g/L or more, 25 g/L or more, 50 g/L or more, 55 g/L or more, 60 g/L or more, 65 g/L or more, 70 g/L or more, 75 g/L or more or 77 g/L or more, for example, 26 g/L or 77 g/L, and the upper limit of the 3-HP concentration is not particularly limited, and for example, it may comprise it at a concentration of 1000 g/L or less, or 100 g/L or less, but not limited thereto.

The fermented composition may comprise 1,3-PDO and 3-HP simultaneously, but not limited thereto.

The fermented composition may be prepared by the microorganism provided in the present description, but not limited thereto.

The fermented composition may be prepared by the method for producing 1,3-PDO and/or 3-HP provided in the present description, but not limited thereto.

### [ADVANTAGEOUS EFFECTS]

The present invention relates to a microorganism to which GPD gene, GPP gene, dhaB, gdrAB, aldH and/or yqhD genes are introduced and/or uses thereof, and has an effect of producing 1,3-PDO and/or 3-HP.

### [BRIEF DESCRIPTION OF THE DRAWINGS]

FIG. 1 is a graph showing the result of confirming production of 1,3-PDO and 3-HP after culturing a microorganism to which dhaB, gdrAB, aldH and/or yqhD genes are introduced.
FIG. 2 is a graph showing the result of confirming production of 1,3-PDO and 3-HP after culturing a microorganism to which GPD gene, GPP gene, dhaB, gdrAB, aldH and/or yqhD genes are introduced.

### [MODE FOR INVENTION]

Hereinafter, the present invention will be described in detail by examples. However, the following examples illustrate the present invention only, but the present invention is not limited by the following examples.

### Example 1. Plasmid design for strain recombination

### Example 1-1. Construction of pCDF-dhaB1,2,3-gdrA,B-aldH-yqhD vectors

A vector comprising yqhD was constructed to overexpress an yqhD gene. Specifically, the yqhD gene (SEQ ID NO: 1) was prepared by amplifying through PCR using a primer pair consisting of the nucleic acid sequences of SEQ ID NO: 2 and SEQ ID NO: 3, from the genome of an E. coli W3110 strain (yqhD, glpK, ldhA, ack-pta, gldA, and ptsG deletion; galP and glk overexpression; named DKALGP:: PK strain; KCCM 40219) (pre-denaturation at 95°C for 2 minutes, denaturation at 95°C for 1 minute, annealing at 55°C for 30 seconds, and elongation at 72°C for 30 seconds, repeated 30 times, final elongation at 72°C for 2 minutes, and then maintaining and storing at 4C , and the final product size: 1.1kb), and information of the yqhD gene and primers was shown in Tables 1 and 2 below.

**[Table 1]**

| Gene name | Sequence (5' -> 3' ) | SEQ ID NO |
|---|---|---|
| yqhD_gene | cccacctctccgactacggtctggacggcagctccatcccggctttgctgaaaaaactggaagagcacggcatgac ccaactgggcgaaaatcatgacattacgttggatgtcagccgccgtatatacgaagccgcccgctaa | 1 |

**[Table 2]**

| Primer type | Sequence (5' -> 3' ) | SEQ ID NO |
|---|---|---|
| yqhD_Primer_For | tactagcgcagcttaatgaacaactttaatctgcacaccc | 2 |
| yqhD_Primer_Rev | cagcagcctaggttaattagcgggcggcttcgtatatacgg | 3 |

The prepared yqhD gene was cloned to the pCDF-dhaB1,2,3-gdrA,B-aldH plasmid which is for producing 3HP.

Specifically, the pCDF-dhaB1,2,3-gdrA,B-aldH plasmid was a vector in which the promoter portion of pCDFDuet-1 vector (Novagen 71340) was substituted with J23101 and J23100 promoters, and dhaB (dhaB1, dhaB2, dhaB3) and gdrAB genes (gdrA, gdrB) of Klebsiella pneumoniae (K. pneumoniae genomic DNA; KCTC12385) and aldH gene derived from E. coli K12 MG1655 were cloned. The dhaB (about 2.7kb; dhaB1, dhaB2, dhaB3) and gdrAB gene (about 2.2kb; gdrA, gdrB) were amplified using the conditions provided from NEB using the primers of Table 3 below and NEB (New England Biolabs) Q5 DNA polymerase in chromosome of Klebsiella pneumonia. In addition, information of the promoters was shown in Table 4 below.

**[Table 3]**

| Primer type | Sequence (5' -> 3' ) | SEQ ID NO |
|---|---|---|
| dhaB-gdrA_Primer_For | GAATTCATGAAAAGATCAAAACGATTTGCAGTCCT | 4 |
| dhaB-gdrA_Primer_Rev | AAGCTTGATCTCCCACTGACCAAAGCTGG | 5 |
| gdrB_Primer_For | AAGCTTAGAGGGGGCCGTCATGTCGCTTTCACCGCCAG | 6 |
| gdrB_Primer_Rev | CTTAAGTCAGTTTCTCTCACTTAACGGC | 7 |

**[Table 4]**

| Promoter type | Sequence (5' -> 3' ) | SEQ ID NO |
|---|---|---|
| J23101_Promo | tttacagctagctcagtcctaggtattatgctagc | 8 |
| J23100_Promo | ttgacggctagctcagtcctaggtacagtgctagc | 9 |

On the chromosome of Klebsiella pneumonia, dhaB123 and gdrA genes were positioned side by side, so they were amplified together, and gdrB was positioned in the opposite direction to dhaB123 and gdrA, so only gdrB was separately amplified, and then, NEB Q5 DNA polymerase was used, and as the condition, the condition provided by NEB was used. After that, the dhaB123, gdrA genes were cloned using restriction enzymes EcoRI and HindIII, and the gdrB gene was cloned using restriction enzymes HindIII and AflII, under the J23101 promoter, and the pCDF-dhaB1,2,3-gdrA,B plasmid (hereinafter, pCDF-dhaB-gdrAB vector) was prepared.

aldH was amplified from E. coli K12 MG1655 chromosome using the J23100 promoter, and then the primers of Table 5 below and NEB Q5 DNA polymerase were used, and the condition provided by NEB was used.

**[Table 5]**

| Primer type | Sequence (5° -> 3' ) | SEQ ID NO |
|---|---|---|
| aldH_Primer_For | ggtaccatgaattttcatcatctggc | 10 |
| aldH_Primer_Rev | catatgtcaggcctccaggcttat | 11 |

aldH was cloned under the J23100 promoter of the pCDF-dhaB-gdrAB vector using restriction enzymes KpniI and NdeI through T4 DNA ligase reaction, and the pCDF-dhaB1,2,3-gdrA,B-aldH plasmid (hereinafter, pCDF-dhaB-gdrAB-aldH vector) was prepared.

To the pCDF-dhaB1,2,3-gdrA,B-aldH plasmid prepared as above, the prepared yqhD gene was cloned using PacI restriction enzyme through In-Fusion reaction, and finally, the recombinant vector pCDF-dhaB1,2,3-gdrA,B-aldH-yqhD was prepared (hereinafter, pCDF-dhaB-gdrAB-aldH-yqhD vector). Additionally, the pCDF-dhaB-gdrAB-yqhD vector in which yqhD instead of aldH was cloned to the pCDF-dhaB-gdrAB vector by the same method was prepared.

### Example 1-2. Construction of pRSF-pLTTR-GPD-GPP vector

For Saccharomyces cerevisiae (BY4741; ATCC 201388)-derived GPD1 and GPP2 genes, PCR (pre-denaturation at 95°C for 2 minutes; denaturation at 95°C for 1 minute, annealing at 55°C for 30 seconds, and elongation at 72°C for 2 minutes, repeated 28 times; final elongation at 72°C for 5 minutes; maintaining and storing at 4°C ) was performed using the primer pair of SEQ ID Nos: 12 and 13 or SEQ ID Nos: 14 and 15 of Table 6 below, respectively, and each of them was cloned to the pRSFDuet-1 vector comprising LTTR promoter (Invitrogen), by treatment of BamHI/SacI (using the primer pair of SEQ ID Nos: 12 and 13) and KpnI/XhoI (using the primer pair of SEQ ID Nos: 14 and 15), and a vector comprising pRSF-pLTTR-GPD-GPP (hereinafter, pRSF-pLTTR-GPD-GPP vector) was constructed. The sequence of the used LTTR promoter was described in Table 7 below.

**[Table 6]**

| Primer type | Sequence (5' -> 3' ) | SEQ ID NO |
|---|---|---|
| GPD1_Primer_For | catcatcatggatccatagcagtaagaaaggagcatccatcatgagcgctgcggctg | 12 |
| GPD1_Primer_Rev | catcatcatgagctctcaatcttcatgaaggtctaattcttcaatcatg | 13 |
| GPP2_Primer_For | catcatcatggtaccacagttttagtaaaggagcatcaaaaatgggactcactacgaaaccg | 14 |
| GPP2_Primer_Rev | catcatcatctcgagttaccatttcagcagatcgtctttgg | 15 |

**[Table 7]**

| Promoter type | Sequence (5' -> 3' ) | SEQ ID NO |
|---|---|---|
| LTTT_Promo | catcatcatggatccatagcagtaagaaaggagcatccatcatgagcgctgcggctg | 16 |

### Example 2. Preparation and culture of transformed strain

### Example 2-1. Preparation of strain

In order to prepare a transformed strain with the vector prepared in Example 1, Escherichia coli W3110 (yqhD, glpK, ldhA, ack-pta, gldA, and ptsG deletion; galP and glk overexpression; named DKALGP:: PK strain) strain was prepared. The strain was streak plated on an LB plate (comprising 10g tryptone, 5g yeast extract, 10g NaCl, 15g agar per 1L), and cultured at 37°C overnight. The cultured single colony was inoculated to a 5 mL LB liquid medium (the medium composition was same as the LB plate, but did not comprise agar), and cultured with stirring at 37°C overnight. The next morning, the culture solution was diluted to 1: 100 in 5 mL NL liquid medium and cultured for 3-6 hours until the OD reached ~0.5. By cooling on ice for 10-15 minutes and then centrifuging at 4°C at 4000 rpm for 10 minutes, cells were collected. The cells were resuspended in 1mL of sterile DI water (Deionized water) cooled with ice and washed, and then centrifuged at 4°C at 4000 rpm for 10 minutes again to collect the cells. The collected cells were resuspended in 1mL of DI water and washed again, and centrifuged at 4°C at 4000 rpm for 10 minutes again to collect the cells. The collected cells were resuspended in 0.08mL of DI water, and cell aliquots were added to an individual pre-chilled microfuge tube.

### Example 2-2. dhaB, gdrAB, aldH and/or yqhD gene transformation

For the collected cells in Example 2-1, the pCDFDuet-1 (pCDF) of Example 1, and the plasmid DNA constructed in Example 1, each of the pCDF-dhaB-gdrAB vector, pCDF-dhaB-gdrAB-yqhD vector and pCDF-dhaB-gdrAB-aldH-yqhD vector were added by pipetting them by 1 µ L and then 0.08 to 0.09 ml of the mixture was transferred to a pre-treated 0.1cm electroporation cuvette (pre-chilled electroporation cuvette).

Electroporation was performed at 1.8kV, and immediately after the pulse, 1 mL of the LB liquid medium of the room temperature was added to the cells and they were cultured at 37°C for 1 hour. The culture solution was plated on an LB plate comprising streptomycin and an excessive amount of liquid was made to be dried and absorbed into the plate. The plate was reversed and cultured at 37°C to prepare a single colony.

### Example 2-3. GPD and GPP gene additional transformation

In addition, for the collected cells in Example 2-1, together with 1 µ L of the plasmid DNA pRSF-pLTTR-GPD-GPP vector prepared in Example 1 (hereinafter, pRSF-GG vector), the pCDFDuet-1 (pCDF) of Example 1, and the plasmid DNA constructed in Example 1, each of the pCDF-dhaB-gdrAB vector, pCDF-dhaB-gdrAB-aldH vector, pCDF-dhaB-gdrAB-yqhD vector and pCDF-dhaB-gdrAB-aldH-yqhD vector (1 µ L) were added by pipetting them, and then 0.08 to 0.09 ml of the mixture was transferred to a pre-treated 0.1cm electroporation cuvette (pre-chilled electroporation cuvette). Electroporation was performed at 1.8kV, and immediately after the pulse, 1 mL of the LB liquid medium of the room temperature was added to the cells and they were cultured at 37°C for 1 hour. The culture solution was plated on an LB plate comprising streptomycin and an excessive amount of liquid was made to be dried and absorbed into the plate. The plate was reversed and cultured at 37°C to prepare a single colony.

### Example 3. Confirmation of produced amounts of 1,3-propanediol (1,3-PDO) and 3-hydroxypropionic acid (3-HP) of strain in which dhaB, gdrAB, aldH and/or yqhD genes are transformed

### Example 3-1. Confirmation of produced amounts of 1,3-propanediol (1,3-PDO) and 3-hydroxypropionic acid of transformed strain through flask culture

A modified M9 medium of 100mL comprising 20g/L glycerol, 5g/L glucose and 25mg/L streptomycin/kanamycin (containing MgSO₄· 7H₂O 0.6g/L, NaCl 1.5g/L, Na₂HPO₄· 7H₂O 12.8g/L, K₂HPO₄ 17.4g/L, NH₄Cl 2g/L, yeast extract 0.5g/L, KH₂PO₄ 3g/L and CaCl₂ 0.11g/L) was prepared in a 250mL Erlenmeyer flask. Then, pH was maintained at 6.0 using Ca(OH)₂.

1mL of the single colony prepared in Example 2-2 was inoculated to the prepared M9 medium flask, and cultured in a 33°C , 250rpm shaking incubator, and the absorbance of the strain culture solution was measured at OD600 using a UV Spectrometer under the analysis conditions of Table 8 below using HPLC, and the produced amounts of 1,3-PDO and 3-HP of the transformed strain of Example 2-2 were confirmed, and the results were shown in Table 9 below.

**[Table 8]**

| HPLC Model | Agilent Technologies 1200 Series |
|---|---|
| Column | Bio-Rad Aminex HPX-87H Ion Exclusion Column300 mmx 7.8 mm |
| Detector | Refractive Index (RI) / Ultraviolet (UV) Detector |
| Mobile Phase | 0.5 mM H₂SO₄ |
| Flow rate | 0.4 mL/min |
| Run time | 35 min |
| Column temperature | 35 °C |
| Detector temperature | 35 °C |
| Injection volume | 10*µℓ* |

**[Table 9]**

| Vector type in transformed strain | 1,3-PDO (g/L) | 3-HP (g/L) |
|---|---|---|
| pCDF | 0 | 0 |
| pCDF-dhaB-gdrAB | 0 | 0 |
| pCDF-dhaB-gdrAB-yqhD | 1.42 | 0 |
| pCDF-dhaB-gdrAB-aldH-yqhD | 2.40 | 1.31 |

As a result, when only the empty vector (pCDF) or dhaB/gdrAB was expressed, both 1,3-PDO and 3-HP were not produced. However, it was confirmed that when dhaB/gdrAB was expressed with yqhD, 1,3-PDO was produced by 1.42 g/L and when aldH and yqhD were simultaneously expressed, 1,3-PDO and 3HP were simultaneously produced. It was confirmed that the total target produced amount increased, as the 1,3-PDO produced amount increased about 70% compared to single production and 3HP was additionally produced during simultaneous fermentation.

### Example 3-2. Confirmation of produced amounts of 1,3-propanediol (1,3-PDO) and 3-hydroxypropionic acid (3-HP) of transformed strain through high-concentration cell culture

The single colony of Example 2-2 was inoculated to an LB liquid medium with the same composition as the LB liquid medium of Example 2-2, and pre-cultured (Seed culture) under the condition of 37°C for 18 hours.

Then, for high-concentration cell culture, in a 5L fermenter, the preculture solution (Seed culture) was inoculated to 2L of a modified MR medium comprising 20g/L glucose, 25mg/L streptomycin, and 50mg/L kanamycin (containing MgSO₄ · 7H₂O 0.8 g/L, (NH₄)₂HPO₄ 4 g/L, KH₂PO₄ 6.67 g/L and Citrate 0.8 g/L) in a 150mL flask, and it was cultured under the conditions of 35°C, 500 rpm, pH 6.95 (adjusted using NH₄(OH)). After exhausting the glucose comprised in the medium, Feeding Solution (comprising glucose 700g/L, MgSO₄ 15g/L, Trace metal 10mL/1L, 10x MR salt 100mL/1L, and 25mg/L streptomycin) was added at a speed of 40 mL/hr and it was cultured for 24 hours.

After high-concentration cell culture, for 3-HP production, the culture solution was inoculated to 2L of a new MR medium (containing (NH₄)₂HPO₄ 4 g/L, KH₂PO₄ 6.67 g/L and Citrate 0.8 g/L) at initial O.D. 25 in the medium and 1 µ M of Vitamin B₁₂ was added to produce 3-HP. Glucose 200 g/L and glycerol 500 g/L Feeding Solution was added at a speed of 35 mL/hr, and Mg(OH)₂ was added to maintain the condition of pH 6.95.

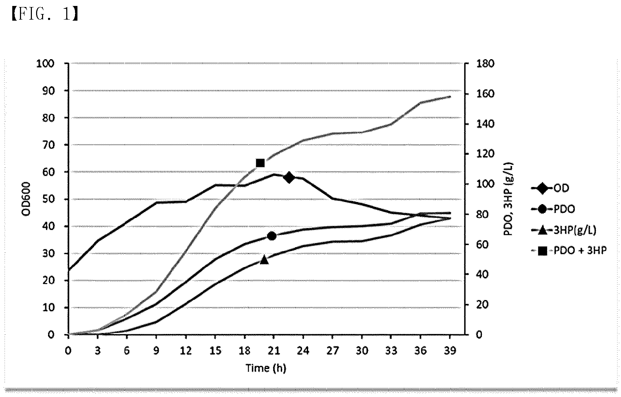

After that, the produced amounts of 1,3-PDO and 3-HP of the strain in which pCDF-dhaB-gdrAB-aldH-yqhD was transformed were confirmed using HPLC by the substantially same method as Example 3-1 above, and the results were shown in FIG. 1 and Table 10. The yield was calculated by the following Equation 1. Yield (%) = (Produced amounts of 1.3-PDO and 3-HP) / (Amount of used glucose and glycerol) * 100

**[Table 10]**

| Type | Measured value |
|---|---|
| 1,3-PDO (g/L) | 81 |
| 3-HP (g/L) | 77 |
| 1,3-PDO productivity (g/L/hr) | 2.1 |
| 3-HP productivity (g/L/hr) | 2.0 |
| Yield (%, g/g) | 63 |

(In Table 10 above, the productivity means the produced amount per hour)

As a result, the high 1,3-PDO and 3-HP produced amounts and productivity were confirmed, and it was confirmed that the yield was also high.

### Example 4. Confirmation of produced amounts of 1,3-propanediol (1,3-PDO) and 3-hydroxypropionic acid (3-HP) of strain transformed with GPD and GPP genes

### Example 4-1. Confirmation of produced amounts of 1,3-propanediol (1,3-PDO) and 3-hydroxypropionic acid (3-HP) of transformed strain through flask culture

A modified M9 medium (containing MgSO₄ · 7H₂O 0.6g/L, NaCl 1.5g/L, NagHPO₄ · 7H₂O 12.8g/L, K₂HPO₄ 17.4g/L, NH₄Cl 2g/L, yeast extract 0.5g/L, KH₂PO₄ 3g/L and CaCl₂ 0.11g/L) of 100 mL comprising 20g/L glycerol, 5g/L glucose and 25mg/L streptomycin/kanamycin was prepared in a 250mL Erlenmeyer flask. Then, using Ca(OH)₂, pH was maintained at 6.0.

The single colony prepared in Example 2-3 of 1mL was inoculated to the prepared M9 medium flask, and cultured in a 33°C, 250rpm shaking incubator, and the absorbance of the strain culture solution was measured at OD600 using a UV spectrometer under the analysis conditions of Table 11 below, and the produced amounts of 1,3-PDO and 3-HP of the transformed strain of Example 2-3 were confirmed, and the results were shown in Table 12 below.

**[Table 11]**

| HPLC Model | Agilent Technologies 1200 Series |
|---|---|
| Column | Bio-Rad Aminex HPX-87H Ion Exclusion Column300 mm x7.8 mm |
| Detector | Refractive Index (RI) / Ultraviolet (UV) Detector |
| Mobile Phase | 0.5 mM H₂SO₄ |
| Flow rate | 0.4 mL/min |
| Run time | 35 min |
| Column temperature | 35 °C |
| Detector temperature | 35 °C |
| Injection volume | 10*µ*ℓ |

**[Table 12]**

| Vector type in transformed strain | 1,3-PDO (g/L) | 3-HP (g/L) |
|---|---|---|
| pRSF-GG, pCDF | 0 | 0 |
| pRSF-GG, pCDF-dhaB-gdrAB | 0 | 0 |
| pRSF-GG, pCDF-dhaB-gdrAB-aldH | 0 | 3.43 |
| pRSF-GG, pCDF-dhaB-gdrAB-yqhD | 1.42 | 0 |
| pRSF-GG, pCDF-dhaB-gdrAB-aldH-yqhD | 5.21 | 2.76 |

As a result, when only the empty vector (pCDF) or dhaB/gdrAB was expressed, both 1,3-PDO and 3HP were not produced. However, it was confirmed that 3HP or 1,3-PDO was produced at 3.43 g/L, 1.42 g/L, respectively, when the dhaB/gdrAB and GPD-GPP were expressed with aldH or yqhD, and 1,3-PDO and 3HP were simultaneously produced, when aldH and yqhD were simultaneously expressed. It was confirmed that the total target produced amount increased, as the 1,3-PDO produced amount increased about 3.7 times compared to single production and 3HP was additionally produced during simultaneous fermentation.

### Example 4-2. Confirmation of produced amounts of 1,3-propanediol (1,3-PDO) and 3-hydroxypropionic acid (3-HP) of transformed strain through high-concentration cell culture

The single colony of Example 2-3 was inoculated to an LB liquid medium of the same composition as the LB liquid medium of Example 2-3, and was pre-cultured (seed culture) under the condition of 37°C for 18 hours.

Then, for high-concentration cell culture, in a 5L fermenter, the preculture solution was inoculated to 2L of a modified MR medium comprising 20g/L glucose, 25mg/L streptomycin, and 50mg/L kanamycin (containing MgSO₄ · 7H₂O 0.8 g/L, (NH₄)₂HPO₄ 4 g/L, KH₂PO₄ 6.67 g/L and Citrate 0.8 g/L) in a 150mL flask, and it was cultured under the conditions of 35°C, 500 rpm, pH 6.95 (adjusted using NH₄(OH)). After exhausting the glucose comprised in the medium, Feeding Solution (comprising glucose 700g/L, MgSO₄ 15g/L, Trace metal 10mL/1L, 10x MR salt 100mL/1L, 25mg/L streptomycin, and 50mg/L kanamycin) was added at a speed of 40 mL/hr and it was cultured for 24 hours.

After high-concentration cell culture, for 3-HP production, the culture solution was inoculated to 2L of a new MR medium (containing (NH₄)₂HPO₄ 4 g/L, KH₂PO₄ 6.67 g/L and Citrate 0.8 g/L) at initial O.D. 25 in the medium and 1 µ M of Vitamin B₁₂ was added to produce 3-HP. Glucose 600 g/L Feeding Solution was added at a speed of 35 mL/hr, and Mg(OH)₂ was added to maintain the condition of pH 6.95.

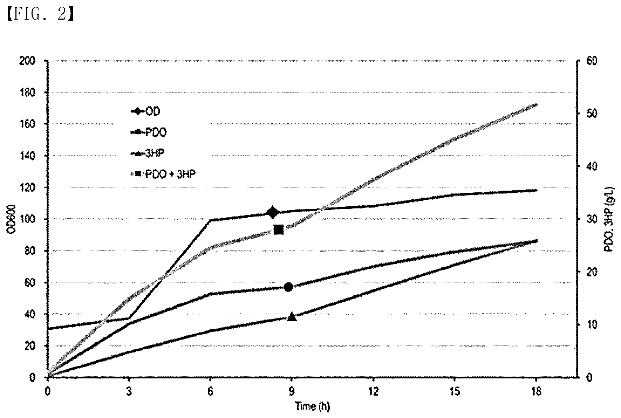

After that, the produced amounts of 1,3-PDO and 3-HP were confirmed using HPLC by the substantially same method as Example 4-1 above, and the results were shown in FIG. 2 and Table 13. The yield was calculated by the following Equation 2. Yield (%) = (Produced amounts of 1.3-PDO and 3-HP) / (amount of used glucose) * 100

**[Table 13]**

| Type | Measured value |
|---|---|
| 1,3-PDO (g/L) | 26 |
| 3-HP (g/L) | 26 |
| 1,3-PDO productivity (g/L/hr) | 1.45 |
| 3-HP productivity (g/L/hr) | 1.45 |
| Yield (%, g/g) | 30 |

(In Table 13 above, the productivity means the total produced amount per hour)

As a result, the high 1,3-PDO and 3-HP produced amounts and productivity were confirmed, and it was confirmed that the yield was also high.

## Claims

1. A microorganism, comprising at least one gene selected from the group consisting of dhaB, gdrAB, aldH and yqhD.

2. The microorganism according to claim 1, further comprising at least one gene selected from the group consisting of a gene encoding glycerol-3-phosphate dehydrogenase (GPD) (GPD gene) and a gene encoding glycerol-3-phosphate phosphatase (GPP) (GPP gene).

3. The microorganism according to claim 1, comprising all of the dhaB, gdrAB, aldH and yqhD genes.

4. The microorganism according to claim 2, comprising both the GPD gene and GPP gene.

5. The microorganism according to claim 1, wherein the microorganism further comprises a characteristic of weakened or inactivated activity of at least one gene selected from the group consisting of yqhD, glpK, ldhA, ack-pta, gldA and ptsG.

6. The microorganism according to claim 2, wherein the microorganism further comprises at least one characteristic selected from the group consisting of the following (a) and (b):
(a) weakened or inactivated activity of at least one gene selected from the group consisting of yqhD, glpK, ldhA, ack-pta, gldA and ptsG; and
(b) enhanced activity of at least one gene selected from the group consisting of galP and glk.

7. The microorganism according to any one claim of claim 1 to claim 6, simultaneously producing 1,3-propanediol (1,3-PDO) and 3-hydroxypropionic acid (3-HP).

8. The microorganism according to any one claim of claim 1 to claim 6, wherein the microorganism is an Escherichia sp. microorganism, a Klebsiella sp. microorganism, or a Lactobacillus sp. microorganism.

9. The microorganism according to any one claim of claim 1, claim 3 and claim 5, producing 1,3-propanediol (1,3-PDO) and 3-hydroxypropionic acid (3-HP) from glycerol.

10. The microorganism according to any one claim of claim 2, claim 4 and claim 6, producing 1,3-propanediol (1,3-PDO) and 3-hydroxypropionic acid (3-HP) from glucose.

11. The microorganism according to any one claim of claim 1 to claim 6,
having 1,3-PDO productivity of 0.5 g/L/hr or more, or
3-HP productivity of 0.5 g/L/hr or more.

12. A recombinant vector, comprising at least one gene selected from the group consisting of dhaB, gdrAB, aldH and yqhD.

13. The recombinant vector according to claim 12, further comprising at least one gene selected from the group consisting of GPD gene and GPP gene.

14. A composition for producing 1,3-PDO and 3-HP, comprising the microorganism according to any one claim of claim 1 to claim 6 or the recombinant vector according to claim 12 or claim 13.

15. A method for producing 1,3-PDO and 3-HP, comprising culturing the microorganism according to any one claim of claim 1 to claim 6.

16. The method for producing 1,3-PDO and 3-HP according to claim 15, wherein the 1,3-PDO productivity of the method for producing 1,3-PDO and 3-HP is 0.5 g/L/hr or more, or
the 3-HP productivity of the method for producing 1,3-PDO and 3-HP is 0.5 g/L/hr or more.

17. A fermented composition, which is obtained by fermenting a microorganism having producing activity of 1,3-PDO and 3-HP, and comprises 1,3-PDO at a concentration of 10 g/L or more, and 3-HP at a concentration of 10 g/L or more.

18. The fermented composition according to claim 17, which is prepared by the microorganism according to any one claim of claim 1 to claim 6.

19. The fermented composition according to claim 17, which is prepared by the method for producing 1,3-PDO and 3-HP according to claim 15.
